# EUROPEAN PATENT SPECIFICATION

(11) **EP 1 586 569 B1**
(45) Date of publication and mention of the grant of the patent: **06.06.2012**
(21) Application number: 05006819.6
(22) Date of filing: 29.03.2005
(51) Int. Cl.: C07D 307/89, C07C 69/80, C07C 63/74

(54) **Aryl ethynyl phthalic acid derivative and method for producing the same**
Aryl Ethinyl Phthalsäurederivate und Verfahren zu deren Herstellung
Dérivés d'acide aryle ethynyle phthalique et procédé de fabrication de ces composés

(30) Priority: 25.03.2004 JP 2004090235; 09.08.2004 JP 2004232675
(43) Date of publication of application: 19.10.2005
(73) Proprietor: FUJIFILM Corporation, Minato-ku Tokyo (JP)
(72) Inventor: Urazoe, Daisuke c/o Fuji Photo Film Co., Ltd., Odawara-shi Kanagawa (JP); Mori, Hideto c/o Fuji Photo Film Co., Ltd., Odawara-shi Kanagawa (JP); Yamakawa, Katsuyoshi c/o Fuji Photo Film Co., Ltd., Odawara-shi Kanagawa (JP)
(74) Representative: HOFFMANN EITLE

(56) References cited:
- US-A- 5 185 454
- US-A- 5 567 800
- DATABASE CAPLUS [Online] CHEMICAL ABSTRACTS SERVICE, COLUMBUS, OHIO, US; BLATCHLY, RICHARD A. ET AL: "Theoretical Study of Helix Formation in Substituted Phenylene Ethynylene Oligomers" XP002339578 retrieved from STN Database accession no. 2003:832966 & JOURNAL OF ORGANIC CHEMISTRY , 68(23), 8780-8785 CODEN: JOCEAH; ISSN: 0022-3263, 2003,
- JOHNSTON J A ET AL: "Synthesis and characterisation of imide oligomers end-capped with 4-(phenylethynyl)phthalic anhydrides" POLYMER, JORDAN HILL, OXFORD, GB, vol. 35, no. 22, October 1994 (1994-10), pages 4865-4873, XP002133875 ISSN: 0032-3861
- PATENT ABSTRACTS OF JAPAN vol. 1999, no. 12, 29 October 1999 (1999-10-29) & JP 11 180970 A (MITSUI CHEM INC), 6 July 1999 (1999-07-06) -& JP 11 180970 A (MITSUI CHEM INC) 6 July 1999 (1999-07-06)
- PATENT ABSTRACTS OF JAPAN vol. 2003, no. 07, 3 July 2003 (2003-07-03) & JP 2003 073372 A (MANAC INC), 12 March 2003 (2003-03-12)
- DATABASE WPI Section Ch, Week 199937 Derwent Publications Ltd., London, GB; Class A41, AN 1999-439454 XP002339610 & JP 11 180970 A (MITSUI PETROCHEM IND CO LTD) 6 July 1999 (1999-07-06)
- TAKEKOSHI T ET AL: "High-temperature thermoset polyimides containing disubstituted acetylene end groups" POLYMER, JORDAN HILL, OXFORD, GB, vol. 35, no. 22, October 1994 (1994-10), pages 4874-4880, XP002133876 ISSN: 0032-3861

## Description

### BACKGROUND OF THE INVENTION

### Field of the Invention

The present invention relates to novel aryl ethynyl phthalic acid derivatives useful for functional materials such as medical and agricultural intermediates and liquid crystal and electronic materials, and to a method for producing the aryl ethynyl phthalic acid derivatives.

### Description of the Related Art

Aryl ethynyl phthalic acid derivatives are important compounds as the starting materials of functional materials such as medical and agricultural intermediates and liquid crystal and electronic materials and, particularly, have recently received attention as subjects of research relating to various types of functional materials which utilize a carbon-carbon triple bond present in a molecule. For example, phenyl ethynyl acid anhydrides are used as terminal end-blocking agents which impart thermal curability, thermal resistance and acid resistance to polyimide oligomers as described in, for example, USP No. 5,567,800; Polymer, vol. 35, pp. 4874 to 4880, 1994 (hereinafter, referred to also as "Non-Patent Document 1"); ibidem, vol. 35, pp. 4857 to 4864, 1994 ("Non-Patent Document 2"); and Functional Materials, vol. 20, No. 12, pp. 33 to 40, 2000. Further, various types of method for producing phenyl ethynyl phthalic anhydrides are described in, for example, JP-A Nos. 11-180970 ("Patent Document 2") and 2003-73372 ("Patent Document 3"), High Performance Polymer, vol. 6, p. 423, 1994; and Polymer Preprints, vol. 35, p. 353, 1995. However, these are not always satisfactory in terms of yield, efficiency or purity.

Accordingly, development of a novel compound in which performance required for a functional material such as a terminal end-blocking agent is improved and of a method for producing a phenyl ethynyl phthalic anhydride with high purity and high yield are in high demand.

Fluorine-containing aryl ethynyl phthalic acid, an alkali metal salt of fluorine-containing aryl ethynyl phthalic acid and fluorine-containing aryl ethynyl phthalic anhydride have been reported in an extremely small number (see, for example, Non-Patent Document 1). Accordingly, in terms of using these compounds as raw materials to provide resin materials with the desired properties, the range of choice is limited.

### SUMMARY OF THE INVENTION

The present invention provides aryl ethynyl phthalic acid derivatives useful for functional materials such as medical and agricultural intermediates and liquid crystal and electronic materials and, in particular, provides aryl ethynyl phthalic acid derivatives used as terminal end blocking materials having superior characteristics useful for these materials. Further, the present invention provides a novel compound which is useful for producing aryl ethynyl phthalic acid derivatives and which is stably produced on an industrial scale, and provides a method for producing the aryl ethynyl phthalic acid derivatives by using the compound.

According to research conducted by the present inventors on phenyl ethynyl phthalic anhydrides, it has been found that the quality of a phenyl ethynyl phthalic anhydride used as a terminal end-blocking material for a polyimide remarkably affects the performance and physical properties of resin materials. For example, it has been found that when a polyimide resin is produced by using phenyl ethynyl phthalic anhydride which is produced by the methods as described in JP-A Nos. 11-180970 and 2003-73372, slightly soluble components may be formed or foam may be formed during thermal molding or curing, so that the resin cannot be stably produced. Namely, the methods that have been reported are not advantageous in terms of the quality assurance of the desired product and the process quality assurance in the post-processing step. Accordingly, a technique which can consistently produce an aryl ethynyl phthalic anhydride with high purity in a substantial quantity has strong been required.

In view of the above, the present inventors conducted intensive research and, as a result, succeeded in synthesizing of a novel compound having a phthalic acid diester structure and found a method for producing an aryl ethynyl phthalic anhydride by using the novel compound, to thereby achieve the present invention.

Namely, the invention is as described below.

A first aspect of the invention provides a method for producing an aryl ethynyl phthalic acid anhydride, as defined in claim 1.

A second aspect of the invention provides an aryl ethynyl phthalic acid diester compound.

In the second aspect, the aryl ethynyl phthalic acid diester compound is represented by the following Formula 3: 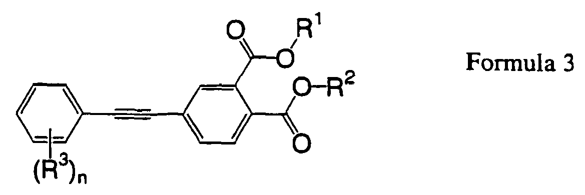 wherein R¹ and R² each independently represent an alkyl group having 1 to 8 carbon atoms, a cycloalkyl group or an aryl group; R³ represents a halogen atom, a hydroxyl group, an alkyl group having 1 to 10 carbon atoms, an aryl group having 6 to 10 carbon atoms, an alkoxy group having 1 to 6 carbon atoms, an aryloxy group having 6 to 12 carbon atoms, an acyl group having 1 to 10 carbon atoms, a perfluoroalkyl group having 1 to 6 carbon atoms or a perfluoroalkoxy group having 1 to 6 carbon atoms; n represents an integer of from 0 to 5; and when n represents 2 or more, such that R³ is present in plurality, each R³ may be the same as or different from any other R³ or linked thereto to form a ring.

In the first aspect, an aryl ethynyl phthalic anhydride represented by the following Formula 5 is produced, wherein an aryl ethynyl phthalic acid diester compound as represented by the following Formula 3 an aryl ethynyl phthalic acid compound represented by the following Formula 4 is hydrolyzed and is subjected to ring-closing: 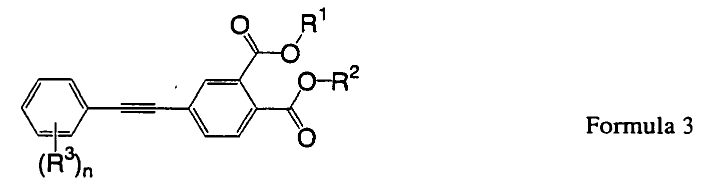 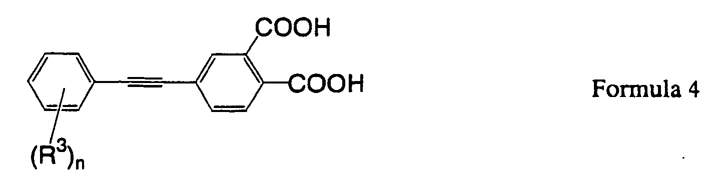 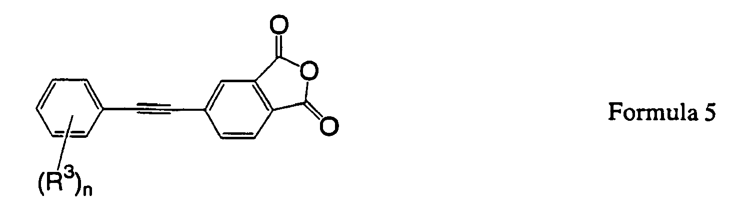 wherein R³ represents a halogen atom, a hydroxyl group, an alkyl group having 1 to 10 carbon atoms, an aryl group having 6 to 10 carbon atoms, an alkoxy group having 1 to 6 carbon atoms, an aryloxy group having 6 to 12 carbon atoms, an acyl group having 1 to 10 carbon atoms, a perfluoroalkyl group having 1 to 6 carbon atoms or a perfluoroalkoxy group having 1 to 6 carbon atoms; n represents an integer of from 0 to 5; and when n represents 2 or more, such that R³ is present in plurality, each R³ may be the same as or different from any other R³ or linked thereto to form a ring, and wherein in Formula 3, R¹ and R² each independently represent an alkyl group having 1 to 8 carbon atoms, a cycloalkyl group or an aryl group.

In the first aspect, the hydrolysis is preferably alkali hydrolysis in which a reaction mixture obtained by hydrolyzing the aryl ethynyl phthalic acid diester compound represented by Formula 3 is subjected to an adsorbent treatment followed by addition of an acid to obtain an aryl ethynyl phthalic acid compound represented by Formula 4.

Preferably, the adsorbent treatment is an adsorbent treatment using active carbon.

### DETAILED DESCRIPTION OF THE INVENTION

Hereinafter, the present invention will be described in detail.

Firstly, an aryl ethynyl phthalic acid diester compound represented by the following Formula 3 of the present invention is described in detail: 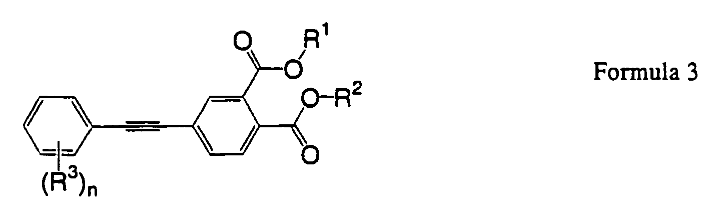 wherein R¹ and R² are as defined in the following and preferable ranges and specific examples of R¹ and R² are as defined in the following

R³ is and the preferable range of R³ is as defined in the following. Preferable examples of R³ include a fluorine atom, a trifluoromethyl group, a tert-butyl group, a tert-butoxy group, a phenyl group, a phenoxy group and a cyano group; n represents an integer of from 0 to 5 and, when n is 2 or more, such that R³ is present in plurality, each R³ may be the same as or different from any other R³ and, further, each R³ may be linked to one another to form a ring (preferably, a carbon ring or a heterocycle). The preferable range of n is from 0 to 2 and, more preferably, 0 or 1.

Examples of R³ include a halogen atom, a hydroxyl group, an alkyl group having 1 to 10 carbon atoms, a cycloalkyl group having 3 to 10 carbon atoms, an aryl group having 6 to 12 carbon atoms, an alkoxy group having 1 to 6 carbon atoms, an aryloxy group having 6 to 12 carbon atoms, an acyl group having 1 to 10 carbon atoms, a perfluoroalkyl group having 1 to 6 carbon atoms, a perfluoroalkoxy group having 1 to 6 carbon atoms, and the like. Among these substituents, a fluorine atom, a hydroxyl group, an alkyl group having 1 to 6 carbon atoms, a cycloalkyl group having 3 to 6 carbon atoms, an alkoxy group having 1 to 6 carbon atoms, an aryl group having 6 to 12 carbon atoms, an aryloxy group having 6 to 12 carbon atoms, a perfluoroalkyl group having 1 to 3 carbon atoms, a perfluoroalkoxy group having 1 to 3 carbon atoms are preferred as substituents and, further, an alkyl group having 1 to 4 carbon atoms, an alkoxy group having 1 to 4 carbon atoms, a phenyl group and a phenoxy group are more preferred. These substituents may be present either singly or in plurality and, when plural substituents are present, they may be the same as or different from one another. Further, these substituents may be bonded with one another to form a carbon ring or a heterocycle.

Specific examples of R³ include a fluorine atom, a hydroxyl group, a methyl group, an ethyl group, a 2-propyl group, a tert-butyl group, a 1-octyl group, a tert-octyl group, a cyclohexyl group, a cyclopentyl group, a cyclopropyl group, a 2-ethylhexyl group, a methoxy group, an ethoxy group, a 2-propoxy group, a tert-butoxy group, a 1-hexyloxy group, a cyclohexyloxy group, a 1-octyloxy group, a 1-naphthyl group, a 2-naphthyl group, a phenyl group, an azulenyl group, a 1-naphthoxy group, a 2-naphthoxy group, a phenoxy group, a pentafluorophenyl group, a 1-pentafluoropropyl group, a trifluoromethyl group, a 1-pentafluoropropoxy group, a trifluoromethoxy group and a pentafluorophenoxy group. Among these substituents, a fluorine atom, a hydroxyl group, an ethyl group, a 2-propyl group, a tert-butyl group, a cyclopropyl group, a cyclohexyl group, a methoxy group, an ethoxy group, a 2-propoxy group, a tert-butoxy group, a cyclohexyloxy group, a 1-naphthyl group, a 2-naphthyl group, a phenyl group, a 1-naphthoxy group, a 2-naphthoxy group, a phenoxy group and trifluoromethoxy group are preferred, and a fluorine atom, a trifluoromethyl group, a tert-butoxy group, a phenyl group and a phenoxy group are more preferred.

R¹ and R² each independently represent an alkyl group, a cycloalkyl group or an aryl group and, preferably, an alkyl group having 1 to 8 carbon atoms, a cycloalkyl group having 3 to 8 carbon atoms and an aryl group having 6 to 12 carbon atoms and, more preferably, an alkyl group having 1 to 6 carbon atoms, a cycloalkyl group having 3 to 6 carbon atoms and an aryl group having 6 to 10 carbon atoms. Specific examples of R¹ and R² include a methyl group, an ethyl group, a 2-propyl group, a 1-butyl group, a 1-octyl group, a tert-butyl group, a cyclohexyl group, a cyclopentyl group, a cyclopropyl group, a 2-ethylhexyl group, a 1-naphthyl group, a 2-naphthyl group, a phenyl group, a 4-toluyl group and a 2-toluyl group. Particularly, a methyl group, an ethyl group, a 2-propyl group, a tert-butyl group and a phenyl group are preferred and a methyl group and an ethyl group are more preferred.

Hereinafter, specific examples of aryl ethynyl phthalic acid diester compounds according to the invention will be described.

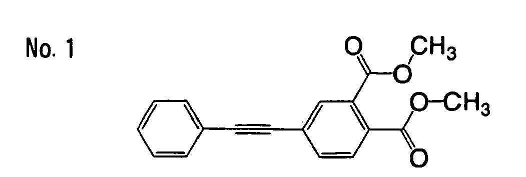

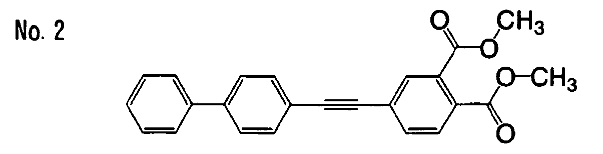

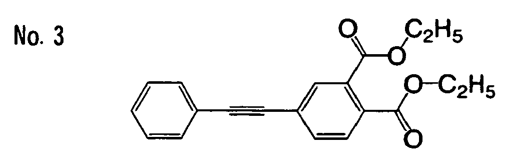

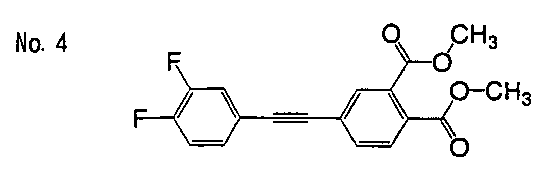

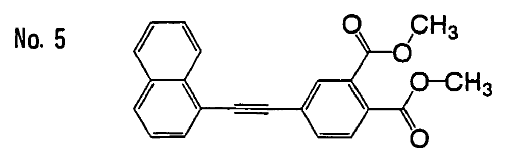

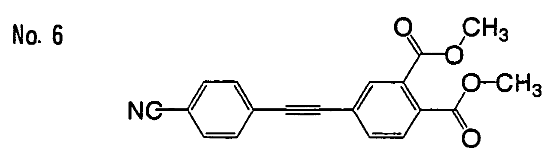

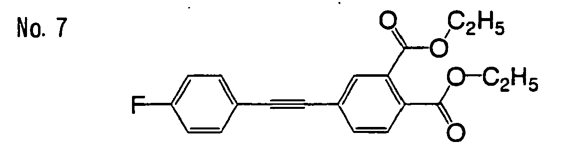

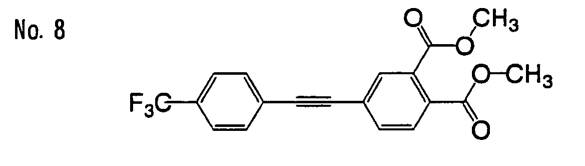

The aryl ethynyl phthalic acid diester compound as represented by Formula 3 can be synthesized by, for example, a method in which a substituted or unsubstituted aryl acetylene compound and a phthalic acid diester having a halogen substituent are coupled in a basic condition by using a palladium (II) complex having copper iodide and a phosphine ligand as a catalyst. According to this method, the aryl ethynyl phthalic acid diester compound as represented by Formula 3 according to the invention can be synthesized in high yield, such that the method is a preferable method. Further, reference may be made to synthetic methods as described in, for example, Journal of Organic Chemistry, vol. 48, pp. 5135 to 5137, 1983 and JP-A No. 10-114691.

Examples of methods for isolating the aryl ethynyl phthalic acid diester compound according to the invention from a reaction mixture include a separation-purification method in which an extraction process is carried out with an organic solvent, followed by chromatography, crystallization, recrystallization or the like. Since the aryl ethynyl phthalic acid diester compound according to the invention has excellent crystallinity, isolation by crystallization is preferred. In a case in which the aryl ethynyl phthalic acid diester compound is crystallized by cooling a solution extracted by the organic solvent, the aryl ethynyl phthalic acid diester compound can be isolated with an ordinary solid-liquid separation. It is also possible that the aryl ethynyl phthalic acid diester compound is crystallized out of a suitable solvent system and, then, isolated by ordinary solid-liquid separation.

Examples of such organic solvents for extracting the aryl ethynyl phthalic acid diester compounds include ether type solvents such as diethyl ether, diisopropyl ether, methyl t-butyl ether and methoxy benzene; ester type solvents such as ethyl acetate and n-butyl acetate; aliphatic hydrocarbon solvents as represented by hexane and heptane; and aromatic hydrocarbon solvents. From the standpoint of applicability to an industrial large-scale production, availability and the like, the ester type solvents, the aliphatic hydrocarbon solvents and the aromatic hydrocarbon solvents are preferred. Examples of organic solvents to be preferably used include toluene, xylene (any one of o-, m- or p-type and mixtures thereof in any mixture ratios are permissible), mesitylene, ethyl benzene, isopropyl benzene (cumene), chlorobenzene, hexane, heptane, ethyl acetate and n-butyl acetate. Among these solvents, toluene, xylene, ethyl benzene, hexane, heptane, ethyl acetate and n-butyl acetate are more favorable and toluene, hexane, heptane and ethyl acetate are still more favorable.

Examples of solvent systems for crystallizing the aryl ethynyl phthalic acid diester compound include a mixed system comprising any one of the above-described organic solvents and any one other organic solvent. Examples of other organic solvents include lower alcohols each having 1 to 4 carbon atoms, acetonitrile and propionitrile. Among these solvents, a solvent to be preferably used can be selected from those lower alcohols having 1 to 4 carbon atoms and acetonitrile. More preferable solvents are methanol, 2-propanol and acetonitrile. Examples of preferable solvents for crystallization include toluene alone, hexane alone, a mixed system of toluene and 2-propanol, a mixed system of hexane and 2-propanol, a mixed system of heptane and 2-propanol, and a mixed system of hexane and acetonitrile. By using any one of the above-described methods, impurities and coloring components attributable to the reaction processes can effectively be removed and, accordingly, the aryl ethynyl phthalic acid diester compound with high purity can be obtained.

Next, a method for producing the aryl ethynyl phthalic acid anhydride according to the invention will be described.

A production method according to the invention is a method in which the aryl ethynyl phthalic acid diester represented by Formula 3 is converted into an aryl ethynyl phthalic acid compound represented by Formula 4 by hydrolysis and, then, the aryl ethynyl phthalic acid compound is subjected to a ring closing reaction to produce an aryl ethynyl phthalic anhydride represented by Formula 5.

As methods of hydrolyzing ester compounds according to the invention, an alkali hydrolysis is preferable. As bases to be used for such alkali hydrolysis, an alkali metal hydroxide, an alkali earth metal hydroxide, an alkali metal alkoxide and an organic salt can be used. A preferable base is an alkali metal hydroxide. Examples of such alkali metal hydroxides include sodium hydroxide, potassium hydroxide, lithium hydroxide and cesium hydroxide. Sodium hydroxide or potassium hydroxide are preferably used. These alkali metal hydroxides may each be used in flake or pellet form or in a solution having an arbitrary concentration (for example, a 25% by mass of aqueous sodium hydroxide solution or a 48 % by mass aqueous potassium hydroxide solution). Taking into account industrial scale production, it is convenient to use the bases in a solution state. An amount of the alkali metal hydroxide to be used in a hydrolysis step is preferably in the range of from 2.0 to 10 times by mole, relative to the aryl ethynyl phthalic acid diester compound represented by Formula 3, , more preferably in the range of from 2.0 to 5.0 times by mole and, still more preferably, in the range of from 2.1 to 3.0 times by mole.

Reaction solvents which can be used in the hydrolysis step are not particularly limited as long as they do not cause an operational problem, do not hinder a reaction and do not give rise to an adverse effect on the reaction due to decomposition in the hydrolysis step in the present invention. However, a mixed system of water and an organic solvent is preferably selected. Preferable examples of organic solvents which can be used in combination with water include alcohol type solvents such as methanol, ethanol and 2-propanol; non-proton type polar solvents such as N-methyl pyrrolidone, sulfolane and N,N-dimethyl imidazolidinone; ether type solvents such as 1,2-dimethoxyethane and tetrhydroxy furan; and nitrogen-containing heteroaromatic type solvents such as pyridine. Further, a plurality of solvents selected from the above-described solvents can be used in combination. Among these solvents, alcohol type solvents, non-proton type polar solvents and pyridine are preferred and methanol, ethanol, 2-propanol, N-methyl pyrrolidone, sulfolane and N, N-dimethyl imidazolidinone are more preferably used. The most preferable solvent is water, methanol, ethanol, N-methyl pyrrolidone or sulfolane, or a mixed system of water and one, two or three types of solvents selected from these solvents.

Reaction temperature in the hydrolysis step is preferably in the range of from 0 to 200°C, more preferably in the range of from 20 to 100°C and, still more preferably, in the range of from 20 to 60°C. Although reaction time period varies according to loading amount and reaction temperature, it is preferably in the range of from 0.5 to 12 hours and, more preferably, in the range of from 1 to 6 hours. As the reaction proceeds, the aryl ethynyl phthalic acid diester compound represented in Formula 3 of the invention is dissolved. In the hydrolysis step, an inert atmosphere is not necessarily required; however, the reaction may be performed in a flow of an argon gas or a nitrogen gas.

An after-treatment of a reaction mixture after completion of the hydrolysis is preferably performed by a method of adsorbent treatment. Examples of such adsorbents include silica gel, magnesium oxide, aluminum oxide, and a commercially available adsorbent comprising a mixed system of these metal oxides, active carbon, zeolite and clay mineral such as montmorillonite. Among these adsorbents, silica gel, a commercially available adsorbent comprising a mixed system of metal oxides, or active carbon is preferable, and a plurality of these adsorbents can be used in combination. In the present invention, the most preferable adsorbent is active carbon. The amount of the adsorbent to be used is preferably in the range of from 0.1 to 200% by mass, relative to the mass of the aryl ethynyl phthalic acid diester compound used as a starting material, , more preferably in the range of from 0.5 to 50% by mass and, still more preferably, in the range of from 1 to 20% by mass. The temperature of the adsorbent treatment is, preferably, in the range of from 0 to 80°C, more preferably in the range of from 10 to 60°C and, still more preferably, in the range of from 20 to 40°C. Although the time period of the adsorbent treatment varies with the loading amount and the temperature, it is preferably in the range of from 0.5 to 12 hours. Further, it is also possible that the reaction mixture in the step of the adsorbent treatment is allowed to stand overnight. The adsorbent after use can easily be removed by ordinary solid-liquid separation. In this process, a filter aid such as Celite, radiolite, cellulose powder and the like may be used.

In the present invention, it is particularly preferable that the adsorbent treatment is performed, followed by converting a salt of the aryl ethynyl phthalic acid into the acid thereof by adding an acid to form the aryl ethynyl phthalic acid represented by Formula 4. Such conversion from the salt of the aryl ethynyl phthalic acid present in the reaction mixture into the salt thereof to form the aryl ethynyl phthalic acid compound as represented by Formula 4 is preferably carried out by allowing an acid to act on a reaction solution after the adsorbent treatment. An amount of the acid to be used is preferably one equivalent or more and, more preferably, 1 to 1.5 equivalents, relative to one carboxylic group,. As types of such acids, acids having a pK value of 4 or less are preferable, more preferably 2.5 or less. These acids include an organic acid such as organic sulfonic acids, for example, methane sulfonic acid, carboxylic acids, for example, formic acid, acetic acid, trichloroacetic acid and citric acid or mineral acids which are inorganic acids such as hydrohalogenic acid, sulfuric acid, nitric acid, phosphoric acid and the like. Of these acids, mineral acids are preferable. Specifically, hydrochloric acid, sulfuric acid, methane sulfonic acid, acetic acid, citric acid and formic acid are preferable. Among these acids, hydrochloric acid, sulfuric acid and methane sulfonic acid are preferable, and hydrochloric acid, sulfuric acid are more preferable. The aryl ethynyl phthalic acid compound represented by Formula 4 is precipitated by carrying out such conversion from the salt to the acid, and a targeted substance can be isolated by performing ordinary solid-liquid separation.

At the time of the conversion into the aryl ethynyl phthalic acid compound as represented by Formula 4 by performing the conversion of the salt to the acid, an organic solvent which can be phase-separated from water in a two layer manner may coexist. This is one of the preferred embodiments according to the present invention. Examples of such solvents which can be phase-separated from water in a two layer manner include ether type solvents such as diethyl ether, diisopropyl ether, methyl t-butyl ether, methoxy benzene and ethoxybenzene; ester type solvents such as ethyl acetate and n-butyl acetate; aliphatic hydrocarbon solvents typically represented by hexane and heptane; and aromatic hydrocarbon solvents. Examples of organic solvents, which are favorably used from the standpoint of applicability to industrial large-scale production, availability and the like, include methyl t-butyl ether, toluene, xylene (any one of o-, m- or p-type and mixtures thereof in any mixture ratios are permissible), mesitylene, ethyl benzene, t-butyl benzene, isopropyl benzene (cumene), chlorobenzene, ethyl acetate, n-propyl acetate and n-butyl acetate. Among these solvents, methyl t-butyl ether, toluene, xylene, ethyl benzene, ethyl acetate, n-butyl acetate are more favorable and methyl t-butyl ether, toluene, xylene and ethyl acetate are still more preferable. A plurality of these solvents can be used in combination. The organic solvent is allowed to coexist with water, so that the aryl ethynyl phthalic acid compound which is prepared by converting the salt into the acid can effectively be extracted in an organic layer. On the other hand, an inorganic salt and the like are transferred into a water layer and removed. The organic layer containing the aryl ethynyl phthalic acid compound separated from the reaction mixture separated into two layers as such can be allowed to proceed to the subsequent ring-closing step. This is industrially advantageous.

A method in which the aryl ethynyl phthalic acid compound as represented by Formula 4 is subjected to ring closing, preferably, chemical or thermal ring closing to derive an aryl ethynyl phthalic anhydride as represented by Formula 5 is not particularly limited. For example, the ring closing step can easily be performed by heating together with acetic anhydride in the presence of a solvent. Alternatively, the ring closing process can be performed by heating at about 110°C to about 150°C. In this case, it is preferable to use a solvent such as toluene having a property capable of removing water from an azeotrope.

Hereinafter, the ring closing method will be described in detail.

A reaction agent is used for performing the ring-closing chemically. The reaction agents include acetic anhydride, succinic anhydride, acetyl chloride, chlorosulfonic acid, thionyl chloride, phosphorus oxychloride and propenyl acetate. Among these reaction agents, acetic anhydride, acetyl chloride, chlorosulfonic acid and phosphorus oxychloride are preferable, and acetic anhydride and phosphorus oxychloride are more preferable.

The reaction solvents are not specifically limited to specific solvents as long as the solvents do not react with an acid anhydride to form an ester or acid amide, such as an alcohol or amine. Examples of such solvents include hydrocarbon-based solvents (including aromatic solvents and aliphatic solvents), halogen-based solvents (including aromatic solvents and aliphatic solvents) amide-based solvents, ester-based solvents, ketone-based solvents, nitril-based solvents, and ether-based solvents. Among these solvents, aromatic hydrocarbon-based solvents, ester-based solvents, ketone-based solvents, nitril-based solvents and ether-based solvents are preferable, and aromatic hydrocarbon-based solvents, ester-based solvents, ketone-based solvents are more preferable.

Specific examples of the solvents include decane, toluene, xylene, chlorobenzene, dimethyl acetoamide, dimethyl formamide, N-methyl-2-pyrrolidone, ethylacetate, butylacetate, diethyl carbonate, cyclohexanone, methyl isobutyl ketone, methyl ethylketone, acetone, acetonitril, propionitril, anisole, t-butyl methylether, diisopropyl ether, and the like. Among these solvents, toluene, xylene, ethylacetate, butylacetate, diethyl carbonate, cyclohexanone, methyl isobutyl ketone, methyl ethylketone, acetone, acetonitril, propionitril, anisole and t-butyl methylether, diisopropyl ether are preferable, and toluene, xylene, ethylacetate, butylacetate, diethyl carbonate, cyclohexanone, methyl isobutyl ketone, methyl ethylketone and acetone are more preferable.

On the other hand, examples of methods of performing ring-closing include a method of utilizing sublimation without a solvent, and a method of using a solvent which has an azeotropic property with water to remove water formed when a ring is closed. Of these, the method using the solvent which has an azeotropic property with water is preferable.

Examples of these solvents include hydrocarbon-based solvents (including aromatic solvents and aliphatic solvents), halogen-based solvents (including aromatic solvents and aliphatic solvents), ester-based solvents, ketone-based solvents and ether-based solvents. Among these solvents, aromatic hydrocarbon-based solvents, ester-based solvents, and ether-based solvents are preferable, and aromatic hydrocarbon-based solvents and ester-based solvents are more preferable.

Specific examples include decane, toluene, xylene, chlorobenzene, butylacetate, diethyl carbonate, cyclohexanone, methyl isobutyl ketone, anisole, 1,2-dimethoxyethane, and the like. Among these solvents, toluene, xylene, butylacetate, diethyl carbonate, anisole, 1,2-dimethoxyethane are preferable, and toluene, xylene, chlorobenzene, butylacetate and diethyl carbonate are more preferable.

In many cases, since an aryl ethynyl phthalic anhydride as represented by Formula 5 is precipitated as a crystal by cooling the reaction solution after the reaction is complete, a targeted substance can be isolated by performing an ordinary solid-liquid separation, or, a poor solvent may be use in combination.. The aryl ethynyl phthalic anhydride to be obtained by such method as described above is highly pure and can favorably be used as a terminal end-blocking agent for, for example, a polyimide resin.

Specific examples of such aryl ethynyl phthalic anhydrides to be favorably produced by the production method according to the invention are as follows:

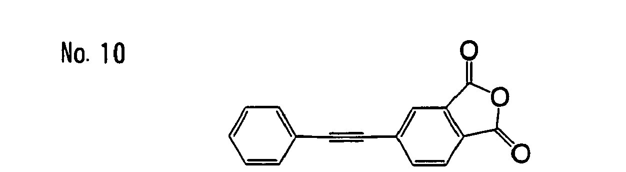

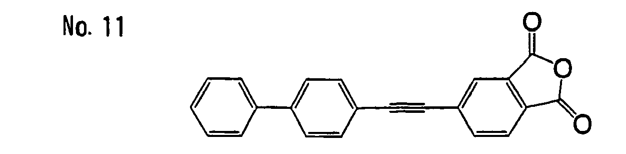

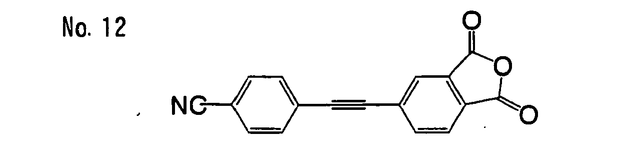

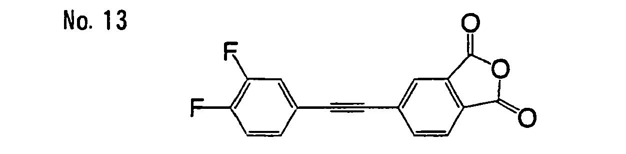

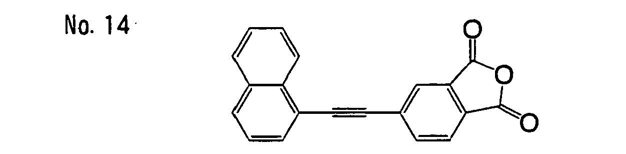

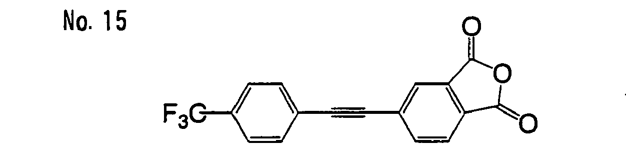

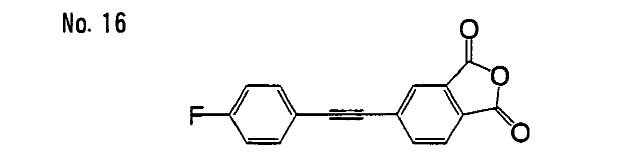

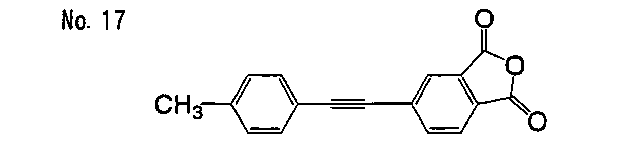

Further examples include :

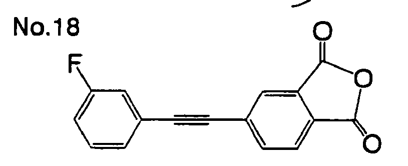

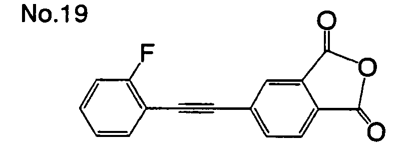

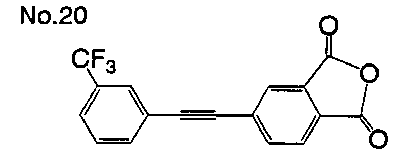

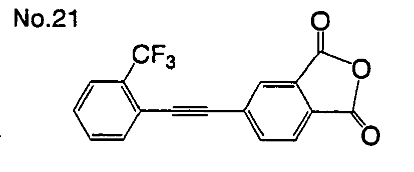

Among the above compounds, the compounds represented by Nos.16 and 18 are most preferable.

In the present invention, the following compounds of formula 4 are preferably used:

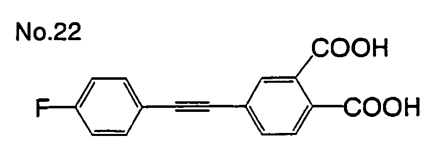

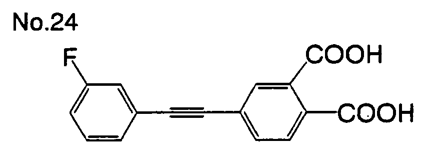

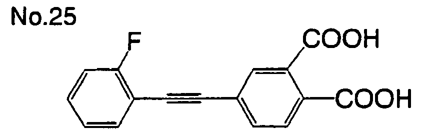

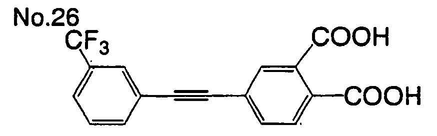

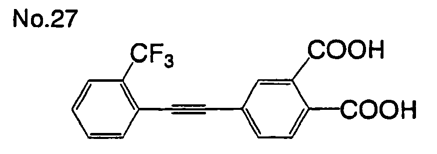

Among these compounds, the compounds Nos. 22 and 24 are preferable.

In the present invention, the fluorine-containing aryl ethynyl phthalic acid or salt thereof are synthesized by subjecting the compounds represented by the above Formula 3, to hydrolysis. The hydrolyzing method is described above.

### EXAMPLES

Hereinafter, the present invention will be described in detail with reference to examples and comparative examples.

### Example 1

### (Compound No. 1: Synthesis of 4-phenyl ethynyl dimethyl phthalate)

Under a nitrogen gas stream, 24.6 g of 4-bromo-dimethyl phthalate, 11.03 g of ethynyl benzene, 71 mg of triphenylphosphine, 20 mg of trans-dichloro-bis (triphenylphosphine) palladium (II) and 171 mg of cuprous iodide were placed in a three-necked flask of 300-ml capacity and, then, 60 ml of triethylamine was poured therein while stirring. The reaction mixture was refluxed for 12 hours while heating at 100°C, cooled to room temperature and, thereafter, the resultant reaction solution was filtered to remove formed salts and catalysts and, then, washed with 60 ml of toluene. The filtrate and a rinsing solution were combined and concentrated , to obtain 25.1 g of a crude crystal of 4-phenyl ethynyl diethyl phthalate.

The thus-obtained crude crystal was recrystallized from a mixed system comprising 2-propanol/n-hexane (50 ml/100 ml), to thereby obtain 22.9 g of 4-phenyl ethynyl diethyl phthalate. The yield thereof was 86.4%.

¹H-NMR (CDCl₃): 3.92 ppm (s, 3H), 3.93 ppm (s, 3H), 7.36 to 7.39 ppm (m, 3H), 7.52 to 7.56 ppm (m, 2H), 7.66 ppm (q, 1H), 7.75 ppm (d, 1H), 7.85 ppm (d, 1H)
IR vmax (KBr): 1269 (s), 1604 (m), 1724 (s), 2210 (w), 2950 (w), 3003 (w) cm⁻¹
MS: m+/z=295
Melting point: 76.9°C to 77.4°C

### Example 2

### Compound No. 4: (Synthesis of 4-(3,4-difluorophenyl-ethynyl) dimethyl phthalate)

A crystal of 4-(3,4-difluorophenyl ethynyl) dimethyl phthalate was obtained in generally the same manner as in Example 1 except for using 11.9 g of 3,4-difluoroethynyl benzene in place of ethynyl benzene. The yield thereof was 68.6%. MS: m+/z=331 and melting point was 96.0°C to 96.7°C.

### Example 3

### (Compound No. 10: Synthesis of 4-phenyl ethynyl phthalic anhydride 10 by using 4-phenyl ethynyl dimethyl phthalate (Straight method without isolation of dicarboxylic acid))

4-phenyl ethynyl dimethyl phthalate (33.6 g) was suspended in a mixed medium comprising water and methanol and a 25% by mass aqueous solution of sodium hydroxide (40 g) was dropwise added thereto with stirring. The resultant reaction mixture was stirred at 60°C for 3 hours and, then, after confirming the completion of the reaction, cooled to the inside temperature of 30°C. Thereafter, 1 g of active carbon was added thereto, and, then, stirred for 30 minutes maintaining the same temperature. The resultant mixture was filtered to remove the active carbon, and rinsed with water. The filtrate and a rinsing solution were combined and, then, toluene and ethyl acetate were added to the resultant mixture. Concentrated hydrochloric acid (28 g) was dropwise added to the resultant 2-layered reaction mixture. The mixture was stirred for 30 minutes at room temperature and was allowed to stand, so that an organic layer containing 4-phenyl ethynyl phthalic acid was separated. After partially concentrating the organic layer, acetic anhydride (17 g) was added thereto, and the reaction mixture was refluxed with heating for 4 hours. After the reaction was completed, the resultant reaction mixture was cooled, so that 4-phenyl ethynyl phthalic anhydride was precipitated as a crystal. The crystal was filtered, rinsed and dried, to thereby obtain 26.6 g of 4-phenyl ethynyl phthalic anhydride as a pale yellow crystal. The yield was 94% on the basis of 4-phenyl ethynyl dimethyl phthalate. Physical properties of the substance thus obtained were as follows:
Melting point: 152.1 to 152.3°C;
IR vmax (KBr): 3070 (w), 2200 (m), 1775 (w), 1770 (s), 1755 (vs), 1620 (s), 1495 (m), 1340 (m), 1240 (vs), 940 (m), 900 (vs) cm⁻¹;
Turbidity: 0.1 ppm (100 mg of sample/25 mL of ethyl acetate solution);
Visible light absorption: 0.015 (400 nm), 0.004 (450 nm) (100 mg of sample/25 mL of ethyl acetate solution); and
GC purity: 99.9% or more (GC measurement conditions: column: DB-5MS, 0.53 mm x 30 m; carrier gas: helium, 70 kPa; detection: FID; column temperature: 100°C to 300°C (temperature being raised at a rate of 10°C/min).

### Example 4

### Compound No. 10: Synthesis of 4-phenyl ethynyl phthalic anhydride 10 by using 4-phenyl ethynyl dimethyl phthalate (a method of isolating dicarboxylic acid)

4-phenyl ethynyl dimethyl phthalate (33.6 g) was suspended in a mixed medium comprising water and methanol and an aqueous 25% by mass sodium hydroxide solution (40 g) was dropwise added to the mixture while being stirred. The resultant reaction mixture was stirred at 60°C for 3 hours and, after confirming the completion of the reaction, cooled to the inside temperature of 30°C. Thereafter, 1 g of active carbon was added thereto, stirred for 30 minutes maintaining the same temperature. The resultant mixture was filtered to remove active carbon and, then, washed with water. The filtrate and a washing solution were combined and, concentrated, hydrochloric acid (28 g) was dropwise added to the resultant mixture. After the resultant reaction mixture was stirred for 30 minutes at room temperature, a precipitated crystal was collected by filtration and, then, the thus-collected crystal was rinsed and dried, to thereby obtain 4-phenyl ethynyl phthalic acid as a colorless crystal (melting point: 211.0 to 211.6°C).

An entire amount of the thus-obtained crystal was suspended in toluene and, acetic anhydride (17 g) was added thereto. The resultant reaction mixture was refluxed with heating for 4 hours. After the reaction was completed, the resultant reaction mixture was cooled, so that 4-phenyl ethynyl phthalic anhydride was precipitated as a crystal. The crystal was filtered, rinsed and dried, to thereby obtain 26.3 g of 4-phenyl ethynyl phthalic anhydride as a pale yellow crystal. The yield was 93% on the basis of 4-phenyl ethynyl dimethyl phthalate. Physical properties of the substance thus obtained were as follows:
Melting point: 152.1 to 152.2°C;
IR: coincided with those in Example 3;
Turbidity: 0.1 ppm (conditions were set to be same as those in Example 3);
Visible light absorption: 0.015 (400 nm), 0.003 (450 nm) (conditions were the same as those in Example 3); and
GC purity: 99.9% or more (measurement conditions were the same as those in Example 3).

### Comparative Example 1

### (Compound No. 10: Synthesis of: 4-phenyl ethynyl phthalic anhydride 10 by using the method as described in the above-described Patent Document No. 3 (JP-A No. 2003-73372))

In accordance with the method as described in Patent Document No. 3, 4-phenyl ethynyl phthalic anhydride was synthesized from 4-bromophthalic anhydride and phenyl acetylene. The resultant 4-phenyl ethynyl phthalic anhydride was yellowish brown crystalline powder. The yield thereof was 79.1 %.

Physical properties were as follows (measurement conditions were the same as those in Example 3):
Melting point: 149.1 to 149.8°C;
Turbidity: 8.1 ppm;
Visible light absorption: 0.058 (400 nm), 0.015 (450 nm); and
GC purity: 97.5%.

### Comparative Example 2

### (Synthesis of 4-phenyl ethynyl phthalic anhydride by using the method as described in the above-described Patent Document No. 2)

In accordance with the method as described in Example 1 of Patent Document No. 2 (JP-A No. 11-180970), 4-phenyl ethynyl phthalic anhydride was synthesized from 4-bromophthalic anhydride and phenyl acetylene. The resultant 4-phenyl ethynyl phthalic anhydride was pale yellow crystalline powder. Physical properties were as follows (measurement conditions were the same as those in Example 3). The yield of the product was 82.3 % :
Melting point: 151.1 to 151.8°C;
Turbidity: 10.5 ppm;
Visible light absorption: 0.050 (400 nm), 0.022 (450 nm); and
GC purity: 98.7%.

### Comparative Example 3

### (Synthesis of 4-phenyl ethynyl phthalic anhydride by using the method as described in Non-Patent Document No. 2)

In accordance with the method as described in Non-Patent Document No. 2 (Polymer, vol. 35, pp. 4858, 1994), 4-phenyl ethynyl phthalic anhydride was synthesized from 4-bromophthalic anhydride and phenyl acetylene. The resultant 4-phenyl ethynyl phthalic anhydride was pale yellow crystalline powder. Physical properties were as follows (measurement conditions were the same as those in Example 3). The yield of the product was 70.6%:
Melting point: 150.5 to 151.1°C;
Turbidity: 12.1 ppm;
Visible light absorption: 0.049 (400 nm), 0.023 (450 nm); and
GC purity: 98.8%.

### Example 5

### (Synthesis of imide oligomer by using 4-phenyl ethynyl phthalic anhydride obtained in Example 3 and Comparative Examples 1 to 3 as terminal end groups)

In accordance with the method as described in Non-Patent Document No. 2, a solution of an amide-acid oligomer having an average molecular weight of about 9,000 was prepared from the 4-phenyl ethynyl phthalic anhydrides obtained in Example 3 and Comparative Examples 1 to 3, and 3, 4'-oxydianiline and an N-methyl pyrrolidone solution of 4,4'-oxydiphthalic anhydride. The thus-prepared amide-acid oligomer was centrifuged, applied, dried and subjected to thermal treatments for one hour at 100°C, 225°C and 350°C in this order, to thereby obtain films of cross-linked imide oligomer. On the other hand, toluene was added to an N-methyl pyrrolidone solution of the amide-acid oligomer and, the mixture was subjected to the steps of azeotropic dehydration, cooling, filtration, rinsing with water and methanol in this order, and drying, to thereby isolate an imide oligomer.

Tg and kinetic properties at 23°C of each film prepared in accordance with the above-described method corresponding to 4-phenyl ethynyl phthalic anhydrides obtained in Example 3 and Comparative Examples 1 to 3 were measured in accordance with a method as specified in ASTM D882 and, also a temperature at which 5% by mass of the imide oligomer was reduced was measured by using a thermobalance. These results are shown in Table 1.

**[Table 1]**

| Production method for 4-phenyl ethynyl phthalic anhydride | Film of cross-linked imide oligomer (23°C) | | | | Temperature of 5% mass reduction |
|---|---|---|---|---|---|
| | Tg | Tensile strength | Elastic modulus | Elongation at break | |
| Example 3 | 252°C | 122.2 MPa | 3.0 GPa | 55% | 518°C |
| Comparative Example 1 | 250°C | 120.1 Mpa | 2.6 GPa | 31% | 514°C |
| Comparative Example 2 | 252°C | 118.8 MPa | 2.8 GPa | 36% | 511°C |
| Comparative Example 3 | 251°C | 119.7 MPa | 2.7 GPa | 36% | 514°C |

As is apparent from Table 1, it is found that the 4-phenyl ethynyl phthalic anhydride obtained by the production method according to the present invention has a high purity and contains an extremely small amount of impurities and the obtained film, by using the 4-phenyl ethynyl phthalic anhydride as a terminal end-blocking material, is excellent so that the film has a high value of tensile strength, elastic modulus, elongation at break and temperature for 5% mass reduction.. Further, when the above-described films were prepared ten times by using the 4-phenyl ethynyl phthalic anhydride obtained in Example 3, generation of a slightly soluble component and foaming at the time of forming or curing were not observed in any of the films. In contrast to the above, when the 4-phenyl ethynyl phthalic anhydrides obtained in Comparative Examples 1 to 3 were used, generation of a slightly soluble component and foaming at the time of forming or curing were sometimes observed.

### Example 6

### (Synthesis of 4-(3-fluorophenylethynyl) dimethyl phthalate)

Under a nitrogen gas stream, 10.9 g of 4-bromo-dimethyl phthalate, 5.8 g of 3-fluoroethynyl benzene, 28 mg of triphenylphosphine, 7.6 mg of trans-dichloro-bis (triphenylphosphine) palladium (II) and 6.2 mg of cuprous iodide were placed in a three-necked flask of 100-ml capacity at room temperature and 20 ml of triethylamine was then poured therein while being stirred. A reaction mixture was heated and refluxed by heating at 120°C for 3 hours, cooled to 70°C and, thereafter, the resultant reaction solution was filtered to remove generated salts and catalysts and, then, rinsed with 20 ml of toluene. The filtrate and a rinsing solution were combined and concentrated to obtain 14.2 g of an oily substance containing 4-(3-fluorophenyethynyl) dimethyl phthalate. The oily substance was recrystallized from a mixed solvent containing 2-propanol/hexane (20ml/400ml), and 9.93g of 4-(3-fluorophenyethynyl) dimethyl phthalate was obtained. The yield thereof was 79.5%.

The physical properties of the product thus obtained were as follows:

¹H-NMR δ(TMS, CDCl₃): 3.92 ppm (s, 3H), 3.93 ppm (s, 3H), 7.05 -7.12 ppm (m, 1H), 7.21 -7.25 ppm (m, 1H), 7.30 -7.38 ppm (m, 2H), 7.66 ppm (dd, 1H), 7.85 ppm (d, 1H)

### Example 7

### (Compound No. 23: Synthesis of 4-(3-fluorophenylethynyl) disodium phthalate)

109.3g of 4-(3-3-fluorophenylethynyl) dimethyl phthalate and 175 ml of water were placed in a three-necked flask of 500 ml capacity to form a suspension, and 123 g of a 25% aqueous sodium hydroxide solution was added dropwise thereto with stirring. The reaction mixture was stirred at 75°C for 3 hours and the completion of the reaction was confirmed. The temperature inside the flask was cooled to 30°C, and 5g of active carbon was added thereto, and the temperature of the mixture was maintained at the same temperature with stirring. The active carbon was removed by filtration by using Celite as a filter aid. After rinsing, the filtrate and a rinsing solution were combined, and 500 ml of 2-propanol was added dropwise thereto over 30 minutes with stirring to precipitate 4-(3-fluorophenylethynyl) disodium phthalate. After stirring in an iced bath for two hours, filtering, washing and drying, 103.4g of a white crystal of 4-(3-fluorophenylethynyl) disodium phthalate was obtained. The yield thereof was 90.0%.

The physical properties of the product thus obtained were as follows:
IR vmax (KBr): 3422 (m), 2362 (w), 2341 (m), 1633 (w), 1576 (vs), 1481 (m), 1420 (s), 1369 (m), 1148 (w), 870 (m), 835 (m), 806(m) cm⁻¹;

### Example 8

### (Compound No. 24: Synthesis of 4-(3-fluorophenylethynyl) phthalic acid)

109.3g of 4-(3-3-fluorophenylethynyl) dimethyl phthalate and 175 ml of water were placed in a three-necked flask of 500 ml capacity to form a suspension, and a 25% aqueous sodium hydroxide solution was added dropwise thereto with stirring. The reaction mixture was stirred at 75°C for 3 hours and the completion of the reaction was confirmed. The temperature inside the flask was cooled to 30°C, and 5g of active carbon was added thereto, and the temperature of the mixture was maintained at the same temperature with stirring. The active carbon was removed by filtration by using Celite as a filter aid. After rinsing, the filtrate and a rinsing solution were combined and 600 ml of ethyl acetate was added thereto, and thereafter 86.3g of concentrated hydrochloric acid was added with heating and stirring. 4-(3-fluorophenylethynyl) phthalic acid was precipitated once, and dissolved. When stirring was stopped, the mixture was separated into two phases, and the water phase was removed. The ethyl acetate solution was concentrated under reduced pressure and removed, so that 114.2g of a crude crystal of 4-(3-fluorophenylethynyl) phthalic acid was obtained. 250 ml of 2-propanol was added to the crude crystal, and placed in a three-necked flask of 1 liter. The temperature inside of the flask was increased to 50°C with stirring, and 500 ml of water was added thereto. Thereafter, the suspension thus formed was cooled to 20°C over 3 hours. After the temperature inside of the flask reached at 20°C, stirring was continued for 1 hour, followed by filtering, washing and drying, to obtain 93.8g of 4-(3-fluorophenylethynyl) phthalic acid was obtained.

The physical properties of the product thus obtained were as follows:
Melting point: 194.4°C (Dehydrated to form 4-(3-fluorophenylethynyl) phthalic anhydride due to heating)
¹H-NMR δ(TMS, dimethyl sulfoxide-d₆): 7.30 -7.36ppm (m, 1H), 7.48 ppm (s, 1H), 7.50 -7.52ppm (m, 2H) 7.60ppm (s, 2H), 7.48 ppm (s, 1H)
IR vmax (KBr): 2885 (m), 2364 (w), 1706 (vs), 1920 (m), 864 (m), 842 (m), 791 (s), 683 (m) cm⁻¹;

### Example 9

### (Compound No. 18: Synthesis of 4-(3-fluorophenylethynyl) phthalic anhydride using 4-(3-fluorophenylethynyl) dimethyl phthalate (Straight method without isolating dicarboxylic acid))

109.3g of 4-(3-3-fluorophenylethynyl) dimethyl phthalate and 175 ml of water were placed in a three-necked flask of 500 ml capacity to form a suspension, and 123g of a 25 % aqueous sodium hydroxide solution was added dropwise thereto with stirring. The reaction mixture was stirred at 75°C for 3 hours and the completion of the reaction was confirmed. The temperature inside the flask was cooled to 30°C and 5g of active carbon was added thereto, and the temperature of the mixture was maintained at the same temperature with stirring. The active carbon was removed by filtration by using Celite as a filter aid. After rinsing, the filtrate and a rinsing solution were combined and 400 ml of methyl ethyl ketone was added, and thereafter 86.3g of concentrated hydrochloric acid was added with heating and stirring. 4-(3-fluorophenylethynyl) phthalic acid was precipitated once, and dissolved. When stirring was stopped, the mixture was separated into two phases, and the water phase was removed. After the organic phase was partially concentrated, 80.1g of acetic anhydride was added thereto and the reaction mixture was heated and refluxed for 4 hours. When the reaction mixture was cooled, 4-(3-fluorophenylethynyl) phthalic anhydride was precipitated as a crystal. The crystal was filtered, washed and dried to obtain 80.2g of a pale yellow crystal of 4-(3-fluorophenylethynyl) phthalic anhydride. The yield thereof was 86.1 % based on 4-(3-3-fluorophenylethynyl) dimethyl phthalate.

The physical properties of the product thus obtained were as follows:
¹H-NMR δ(TMS, CDCl₃): 7.11 -7.18 ppm (m, 1H), 7.26 -7.29 ppm (s, 1H), 7.36 -7.40 ppm (m, 2H), 8.00 ppm (s, 2H), 8.13 ppm (s, 1H)
IR vmax (KBr): 3065 (w), 2352 (w), 2212 (w), 1850 (s), 1778 (vs), 1661 (s), 1576 (m), 1427 (m), 1327 (m), 1250 (vs), 920 (s), 885 (m), 737 (s) cm⁻¹;
Turbidity: 0.1ppm (measurement conditions are the same as those of Example 3)
GC purity: 99.9 % or more (GC conditions: column; (manufactured by J&W Scientific) DB-5MS; 0.53 mm X 30 m; carrier gas, helium 70kPa; sprit ratio, 1:40; detection, FID, column temperature, 100°C → 300°C; temperature increase (10°C/min.)

### Example 10

### (Compound No. 18: Synthesis of 4-(3-fluorophenylethynyl) phthalic anhydride using 4-(3-fluorophenylethynyl) phthalic acid)

85.3g of 4-(3-3-fluorophenylethynyl) phthalic acid, 200 ml of toluene and 45.9g of acetic anhydride were placed in a three-necked flask of 500 ml capacity in this order, and the mixture heated at 120°C with stirring to form a suspension. After it was confirmed that reflux started at the temperature of 114°C in the suspension, the reflux was continued for one hour to proceed the reaction. The solution, in which the starting materials were dissolved, formed by the reaction was cooled to 3°C over 3 hours to precipitate the reaction product. The precipitate was filtered, washed and dried to obtain 70.2g of an extremely pale yellow crystal of 4-(3-fluorophenylethynyl) phthalic anhydride. The physical properties of the product were as follows:
Melting point; 142.9°C
NMR and IR data: coincided with the data obtained in those of Example 9;
Turbidity; 0.4 ppm (measurement conditions were the same as those of Example 3; and
GC purity; 99.9% or more (measurement conditions were the same as those of Example 9.

### Example 11

### (Compound No. 9: Synthesis of 4-(3-fluorophenylethynyl) dimethyl phthalate)

136.54g of 4-bromodimethyl phthalate, 69.7g of 4-ethynyl toluene, 354mg of triphenylphosphine, 94.8mg of trans-dichloro-bis(triphenyphosphine) paradium (II) and 771mg of cuprous iodide were placed in a three-necked flask of 1 liter capacity, and 101.2g of triethylamine was further added while stirring. The reaction mixture was refluxed by heating at 110°C while being stirred for 6 hours, and the temperature inside the flask was cooled to 60°C. The reaction solution was filtered to remove the catalysts and the formed salt, and washed with 200 ml of toluene. The filtrate and a rinsing solution were combined and concentrated to obtain 25.1g of a crude crystal of 4-(4-methylphelethynyl) dimethyl phthalate. The crude crystal was recrystallized from a mixed solvent containing 2-propanol and n-hexane (1/2 by volume) to obtain 133.8g of 4-(4-methylphelethynyl) dimethyl phthalate. The yield of the product was 86.8 %.

The physical properties of the product thus obtained were as follows:
¹H-NMR δ(TMS, CDCl₃): 2.38 ppm (s, 3H), 3.92 ppm (s, 3H), 3.93 ppm (s, 3H), 7.18 ppm (d, 2H), 7.43 ppm (d, 2H), 7.64 ppm (dd, 1H), 7.74 ppm (d, 1H), 7.83 ppm (d, 1H).

### Example 12

### (Compound No. 17: Synthesis of 4-(4-methylphenylethynyl) phthalic anhydride using 4-(4-methylphenylethynyl) dimethyl phthalate (method of isolating dicarboxylic acid)

123.6g of 4-(4-methylphenylethynyl) dimethyl phthalate was suspended in 200g of water, and 141g of an aqueous 25% by mass sodium hydroxide solution was added dropwise thereto while being stirred. The reaction mixture was stirred at 65°C for 2 hours. After confirming the completion of the reaction, the temperature inside the flask was cooled to 30°C and 10g of active carbon was added thereto. The reaction mixture was stirred at the same temperature for 30 minutes, and filtered to remove the active carbon. The filtrate and a rinsing solution were combined, and 98.6g of concentrated hydrochloric acid (35.5% by mass) was added dropwise thereto. 500ml of methyl ethylketone was added to the reaction mixture, and the mixture was heated at 60°C for 30 minutes, and allowed to stand. The water phase was removed and concentrated to obtain 111g of a crude crystal of 4-(4-methylphenylethynyl) dimethyl phthalic acid. This product was recrystallized from a mixed solvent containing 2-propanol and distilled water (1/4 by volume) to obtain 95.63g of a colorless crystal of 4-(4-methylphenylethynyl) phthalic acid. 84g out of the crystal thus obtained was suspended in toluene, and 46g of acetic anhydride was added thereto, and the resultant reaction mixture was refluxed by heating for 2 hours. After termination of the reaction, the mixture was cooled to precipitate a crystal of 4-(4-methylphenylethynyl) phthalic anhydride. The crystal was filtered, washed and dried to obtain 71.6g of a pale yellowish green crystal of 4-(4-methylphenylethynyl) phthalic anhydride. The yield of the product was 77.6 % based on 4-(4-methylphenylethynyl) dimethyl phthalate.

The physical properties of the obtained product were as follows:
¹H-NMR δ(TMS, CDCl₃): 2.40 ppm (s, 3H), 7.20 ppm (d, 2H), 7.45 ppm (dd, 2H), 7.96 ppm (s, 1H), 7.96 ppm (s, 1H), 8.07 ppm (t, 1H)
IR vmax (KBr): 2920 (w), 2214 (w), 1840 (w), 1776 (vs), 1611 (m), 1508 (w), 1321 (m), 1246 (s), 930 (m), 889 (s), 818 (s), 737 (s), 671 (m), 523 (m) cm⁻¹
Turbidity: 0.0ppm (measurement conditions are the same as those of Example 3)
Visible light absorbance: 0.153 (400nm); 0.001 (450nm) (measurement conditions are the same as those of Example 3)
GC purity: 99.9 %

## Claims

1. A method for producing an aryl ethynyl phthalic anhydride represented by the following Formula 5, wherein an aryl ethynyl phthalic acid diester compound as represented by the following Formula 3 an aryl ethynyl phthalic acid compound represented by the following Formula 4 is hydrolyzed and is subjected to ring-closing: 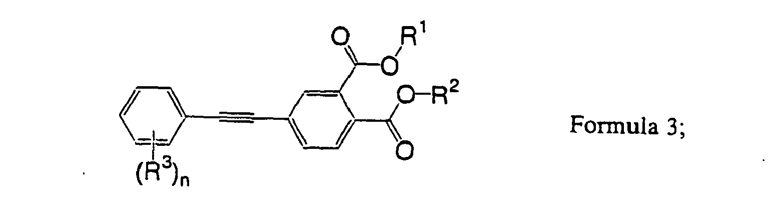 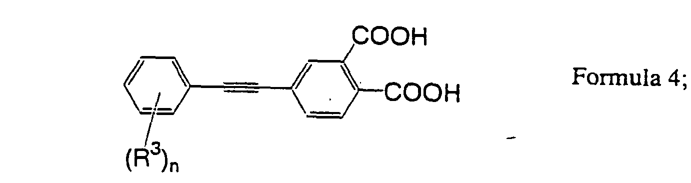 and 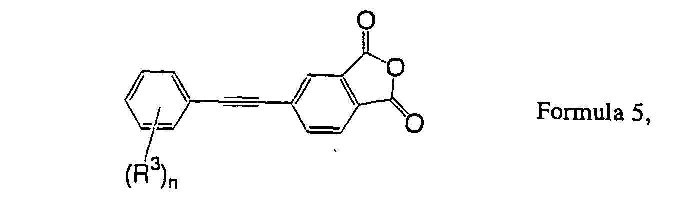 wherein R³ represents a halogen atom, a hydroxyl group, an alkyl group having 1 to 10 carbon atoms, an aryl group having 6 to 10 carbon atoms, an alkoxy group having 1 to 6 carbon atoms, an aryloxy group having 6 to 12 carbon atoms, an acyl group having 1 to 10 carbon atoms, a perfluoroalkyl group having 1 to 6 carbon atoms or a perfluoroalkoxy group having 1 to 6 carbon atoms; n represents an integer of from 0 to 5; and when n represents 2 or more, such that R³ is present in plurality, each R³ may be the same as or different from any other R³ or linked thereto to form a ring and wherein, in Formula 3, R¹ and R² each independently represent an alkyl group having 1 to 8 carbon atoms, a cycloalkyl group or an aryl group.

2. A method according to claim 1, wherein the hydrolysis is alkali hydrolysis in which a reaction mixture obtained by hydrolyzing the aryl ethynyl phthalic acid diester compound represented by Formula 3 is subjected to an adsorbent treatment followed by addition of an acid to obtain an aryl ethynyl phthalic acid compound represented by Formula 4.

3. A method according to claim 2, wherein the adsorbent treatment is an adsorbent treatment using active carbon.

4. An aryl ethynyl phthalic acid diester compound, represented by the following Formula 3: 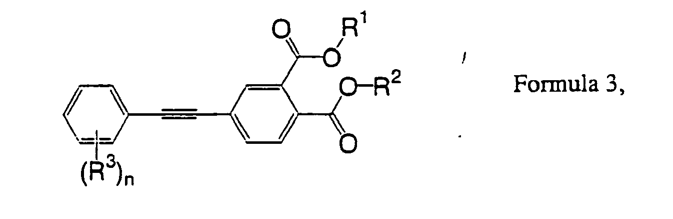 wherein R¹ and R² each independently represent an alkyl group having 1 to 8 carbon atoms, a cycloalkyl group or an aryl group; R³ represents a halogen atom, a hydroxyl group, an alkyl group having 1 to 10 carbon atoms, an aryl group having 6 to 10 carbon atoms, an alkoxy group having 1 to 6 carbon atoms, an aryloxy group having 6 to 12 carbon atoms, an acyl group having 1 to 10 carbon atoms, a perfluoroalkyl group having 1 to 6 carbon atoms or a perfluoroalkoxy group having 1 to 6 carbon atoms; n represents an integer of from 0 to 5; and when n represents 2 or more, such that R³ is present in plurality, each R³ may be the same as or different from any other R³ or linked thereto to form a ring.

## Patentansprüche

1. Verfahren zur Herstellung eines Arylethinyl-Phthalsäureanhydrids, dargestellt durch die folgende Formel 5, worin eine Arylethinyl-Phthalsäurediesterverbindung, wie durch die folgende Formel 3 dargestellt, hydrolysiert und eine Arylethinyl-Phthalsäureverbindung, dargestellt durch die folgende Formel 4, einem Ringschluss unterzogen wird: 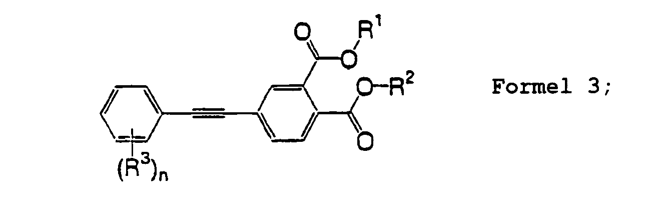 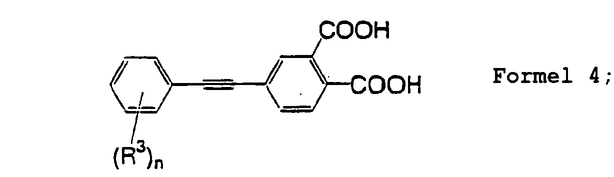 und 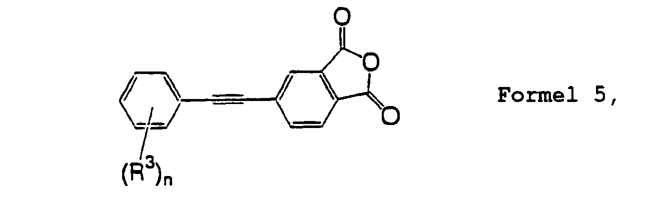 worin R³ ein Halogenatom, eine Hydroxylgruppe, eine Alkylgruppe mit 1 bis 10 Kohlenstoffatomen, eine Arylgruppe mit 6 bis 10 Kohlenstoffatomen, eine Alkoxygruppe mit 1 bis 6 Kohlenstoffatomen, eine Aryloxygruppe mit 6 bis 12 Kohlenstoffatomen, eine Acylgruppe mit 1 bis 10 Kohlenstoffatomen, eine Perfluoralkylgruppe mit 1 bis 6 Kohlenstoffatomen oder eine Perfluoralkoxygruppe mit 1 bis 6 Kohlenstoffatomen darstellt; n eine ganze Zahl von 0 bis 5 darstellt; und wenn n 2 oder größer ist, so dass mehrere R³ vorliegen, jedes R³ gleich oder verschieden von jeglichem anderen R³ sein kann oder hieran gebunden sein kann, um einen Ring zu bilden, und worin in Formel 3 R¹ und R² jeweils unabhängig voneinander eine Alkylgruppe mit 1 bis 8 Kohlenstoffatomen, eine Cycloalkylgruppe oder eine Arylgruppe darstellen.

2. Verfahren gemäß Anspruch 1, worin die Hydrolyse eine Alkalihydrolyse ist, worin eine Reaktionsmischung, erhalten durch Hydrolysieren der durch die Formel 3 dargestellten Arylethinyl-Phthalsäurediesterverbindung, einer Adsorbensbehandlung unterzogen wird, gefolgt von der Zugabe einer Säure, um eine durch die Formel 4 dargestellte Arylethinyl-Phthalsäureverbindung zu erhalten.

3. Verfahren gemäß Anspruch 2, worin die Adsorbensbehandlung eine Adsorbensbehandlung unter Verwendung von Aktivkohle ist.

4. Arylethinyl-Phthalsäurediesterverbindung, dargestellt durch die folgende Formel 3: 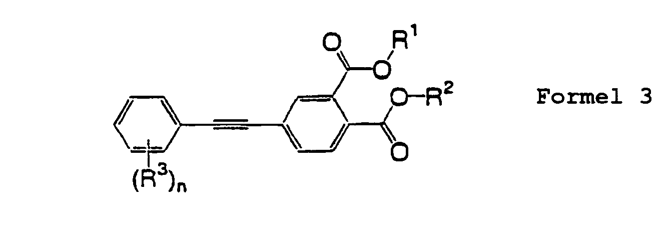 worin R¹ und R² jeweils unabhängig voneinander eine Alkylgruppe mit 1 bis 8 Kohlenstoffatomen, eine Cycloalkylgruppe oder eine Arylgruppe darstellen; R³ ein Halogenatom, eine Hydroxylgruppe, eine Alkylgruppe mit 1 bis 10 Kohlenstoffatomen, eine Arylgruppe mit 6 bis 10 Kohlenstoffatomen, eine Alkoxygruppe mit 1 bis 6 Kohlenstoffatomen, eine Aryloxygruppe mit 6 bis 12 Kohlenstoffatomen, eine Acylgruppe mit 1 bis 10 Kohlenstoffatomen, eine Perfluoralkylgruppe mit 1 bis 6 Kohlenstoffatomen oder eine Perfluoralkoxygruppe mit 1 bis 6 Kohlenstoffatomen darstellt; n eine ganze Zahl von 0 bis 5 darstellt; und wenn n 2 oder größer ist, so dass mehrere R³ vorliegen, jedes R³ gleich oder verschieden von jeglichem anderen R³ sein kann oder hieran gebunden sein kann, um einen Ring zu bilden.

## Revendications

1. Procédé pour produire un anhydride phthalique éthynyle aryle représenté par la Formule suivante 5, dans lequel un composé de diester d'acide phthalique éthynyle aryle représenté par la Formule suivante 3 est hydrolysé et un composé d'acide phthalique éthynyle aryle représenté par la Formule suivante 4 est soumis à une fermeture de cycle : 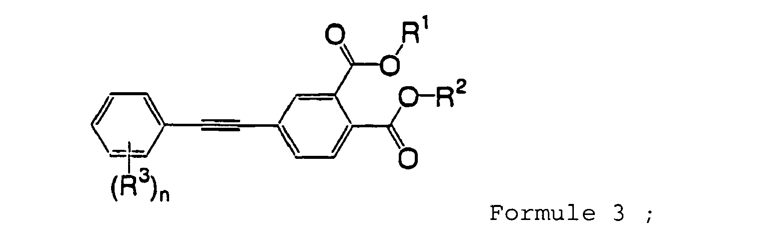 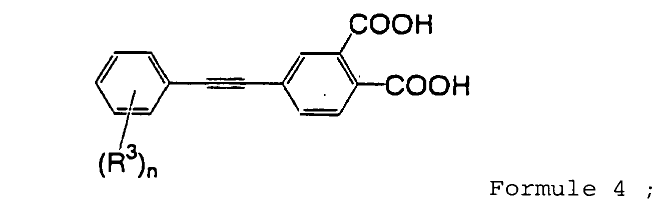 et 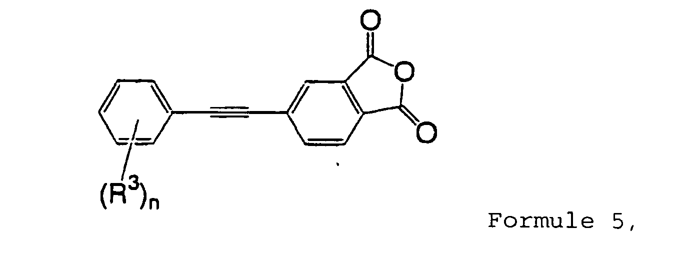 dans lesquelles R³ représente un atome d'halogène, un groupe hydroxyle, un groupe alkyle ayant de 1 à 10 atomes de carbone, un groupe aryle ayant de 6 à 10 atomes de carbone, un groupe alcoxy ayant de 1 à 6 atomes de carbone, un groupe aryloxy ayant de 6 à 12 atomes de carbone, un groupe acyle ayant de 1 à 10 atomes de carbone, un groupe perfluoroalkyle ayant de 1 à 6 atomes de carbone ou un groupe perfluoroalkoxy ayant de 1 à 6 atomes de carbone ; n représente un entier de 0 à 5 ; et quand n représente 2 ou plus, de sorte que R³ est présent en pluralité, chaque R³ peut être le même ou différent de tout autre R³ ou lié à celui-ci pour former un anneau et dans lequel, dans la Formule 3, R¹ et R² représentent chacun indépendamment un groupe alkyle ayant de 1 à 8 atomes de carbone, un groupe cycloalkyle ou un groupe aryle.

2. Procédé selon la revendication 1, dans lequel l'hydrolyse est de l'hydrolyse alcaline dans laquelle un mélange de réaction obtenu en hydrolysant le composé de diester d'acide phthalique éthynyle aryle représenté par la Formule 3 est soumis à un traitement adsorbant suivi par l'ajout d'un acide pour obtenir un composé d'acide phthalique éthynyle aryle représenté par la Formule 4.

3. Procédé selon la revendication 2, dans lequel le traitement adsorbant est un traitement adsorbant utilisant du carbone actif.

4. Composé de diester d'acide phthalique éthynyle aryle, représenté par la Formule suivante 3 : 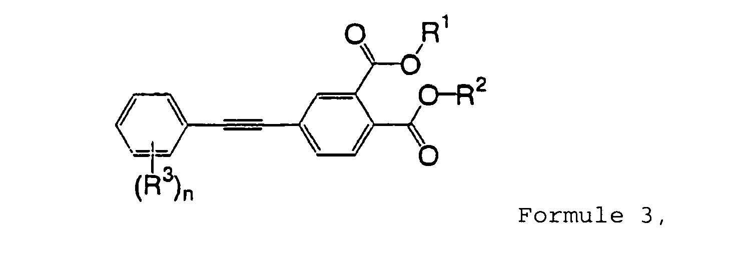 dans laquelle R¹ et R² représentent chacun indépendamment un groupe alkyle ayant de 1 à 8 atomes de carbone, un groupe cycloalkyle ou un groupe aryle ; R³ représente un atome d'halogène, un groupe hydroxyle, un groupe alkyle ayant de 1 à 10 atomes de carbone, un groupe aryle ayant de 6 à 10 atomes de carbone, un groupe alcoxy ayant de 1 à 6 atomes de carbone, un groupe aryloxy ayant de 6 à 12 atomes de carbone, un groupe acyle ayant de 1 à 10 atomes de carbone, un groupe perfluoroalkyle ayant de 1 à 6 atomes de carbone ou un groupe perfluoroalkoxy ayant de 1 à 6 atomes de carbone ; n représente un entier de 0 à 5 ; et quand n représente 2 ou plus, de sorte que R³ est présent en pluralité, chaque R³ peut être le même ou différent de tout autre R³ ou lié à celui-ci pour former un anneau.
